# EUROPEAN PATENT SPECIFICATION

(11) **EP 3 099 219 B1**
(45) Date of publication and mention of the grant of the patent: **15.01.2020**
(21) Application number: 14880874.4
(22) Date of filing: 29.01.2014
(51) Int. Cl.: H04W 4/38, H04W 4/80, A61B 5/00, G06K 19/00

(54) **SYSTEM FOR ESTABLISHING A WIRELESS CONNECTION**
SYSTEM ZUR HERSTELLUNG EINER DRAHTLOSEN VERBINDUNG
SYSTÈME POUR ÉTABLIR D'UNE CONNEXION SANS FIL

(43) Date of publication of application: 07.12.2016
(73) Proprietor: Reaston, Mary, Tulsa, OK 74133 (US); Reaston, Phil, Tulsa, OK 74133 (US)
(72) Inventor: Reaston, Mary, Tulsa, OK 74133 (US); Reaston, Phil, Tulsa, OK 74133 (US)
(74) Representative: Cabinet Chaillot
(86) International application number: PCT/US2014/013645
(87) International publication number: WO 2015/116067

(56) References cited:
- US-A1- 2004 253 923
- US-A1- 2009 023 391
- US-A1- 2009 062 626
- US-A1- 2009 240 814
- US-A1- 2010 256 593
- US-A1- 2011 118 694
- US-A1- 2011 224 503
- US-A1- 2011 224 503
- US-A1- 2012 003 933
- US-A1- 2013 092 728

## Description

### TECHNICAL FIELD

The invention generally pertains to wireless communication, and more particularly to system for establishing a wireless data connection between wireless enabled devices.

### BACKGROUND ART

In the Twenty-first century wireless connectivity has advanced to the stage of being the preferred method of connection for many of the most widely used electronic devices. The most ubiquitous wireless connections today are utilized for telephones and computers, although other devices such as medical diagnostic devices also benefit from wireless connectivity.

Currently, there are three predominant types of wireless connections: BLUETOOTH®, ZigBee and WiFi. Using BLUETOOTH®, a connection protocol known as pairing can be performed to establish a wireless connection. Wireless pairing does have inherent problems and limitations. Pairing is often time consuming as it requires wireless devices to be pre-paired before use. Pairing may also present a security risk since it is possible for a person to insert a non-authorized or unwanted device into the wireless connection.

What is needed is more technologically advanced wireless connection that does not require pairing. An advanced wireless connection would be easier and faster to use, could provide a secure connection and could provide increased functionally such as allowing the concurrent use of multiple wireless devices and the ability to switch out devices while maintaining the wireless connection.

A search of the prior art did not disclose any literature or patents that read directly on the claims of the instant invention. However, the following U.S. patents are considered related:

| **PATENT NO.** | **INVENTOR** | **ISSUED** |
|---|---|---|
| 7,706,794 | Young | April 27, 2010 |
| 7,912,017 | Horisawa | March 22, 2011 |
| 8,295,769 | Bloebaum | October 23, 2012 |
| 8,489,082 | Kaisha | July 16, 2013 |

The 7,706,794 patent discloses a method for connecting personalized devices through a wireless link. In the method, wireless connection information of a plurality of coordinator devices connected to a personalized device through a wireless link is stored. A channel is then set to receive the connection information which is received from a coordinator device through a set channel. If the coordinator device transmitting the connection information is the coordinator to connect, the coordinator device is connected. If not, a coordinator device having the next highest priority is utilized.

The 7,912.017 patent discloses wireless connection system having at least one host apparatus and at least one cheat apparatus. Wireless communication is executed when a host apparatus and a client apparatus execute wireless communication with each other on the basis of a predetermined wireless communication standard. An authentication process is executed for authenticating the connection setting information. If authentication is successful, execution of the wireless communication is enabled.

The 8,295,769 patent discloses a method and a system for wirelessly connecting two data devices. A user intending to connect the devices issues a connect activation command and then moves one device toward the other device along a connection vector. A wireless communication connection is established through Ultra Wideband (UWB) protocol when the two devices are in range of each other.

The 8,489,082 patent discloses a computer program product that comprises computer readable instructions that cause a computer to be wirelessly connectable with at least one device to execute a wireless connection setting process. The wireless connection setting process establishes a wireless connection with the at least one device, transmits externally inputted settings of the wireless connection to the at least one device, and configures the same settings as the settings transmitted to the at least one device.

The 2008000210769 publication application discloses a method and a system for wirelessly connecting two data devices. A user intending to connect the devices issues a connect activation command and then moves one device toward the other device along a connection vector. A wireless communication connection is established through Ultra Wideband (UWB) protocol when the two devices are in range of each other.

US patent application 2011/118694A1 discloses a dispensing system which includes a dispensing unit to dispense therapeutic fluid, and a validation mechanism to enable operation of the dispensing unit, based, at least in part, on a determination of wether operation of the dispensing device is authorized, wherein a remote control unit communicates with a dispensing patch unit.

US patent application 2011/224503A1 discloses an electrodiagnostic functional assessment unit that is designed to wirelessly monitor muscle group activity and to provide treatment for humans and animals, wherein the electrodiagnostic functional assessment is operated by proprietary software which provides testing protocols by utilizing a set of wireless sensors.

US patent application 2013/092728A1 discloses a device agent including an information accessor for accessing association information obtained via an information reader. The association information includes medical device information for uniquely identifying the medical device, and device agent information for facilitating in an association between the medical device and the information reader. The device agent also includes an associator for associating the medical device and the information reader based on the association information.

### DISCLOSURE OF THE INVENTION

The invention is defined by a system for medical assessment according to claim 1. Further embodiments are defined in the dependent claims.

In its most basic design, the system for establishing a wireless connection functions in combination with a first wireless enabled device and at least one second wireless enabled device. The system functions with a conventional wireless communication protocol with as BLUETOOTH®. Zigbee or WiFi, and utilizes a software program that facilitates the first wireless enabled device identifying and establishing a wireless connection with the at least one second wireless device. The system allows a direct connection that does not require a pre-connection discovery function and can provide a more secure connection than other conventional connection methods.

In view of the above disclosure, the primary object of the invention is to provide a system for establishing a wireless connection that does not require a pairing function which is utilized by some wireless communication methods.

In addition to the primary object of the invention, it is also an object of the invention to provide a system for establishing a wireless connection that:
- is easy to use,
- can be utilized with various wireless communication methods
- allows interchangeability of wireless devices,
- can be used with medical devices that require multiple wireless transmission means,
- requires less set-up time than other conventional wireless systems,
- when used with wireless sensors, allows quick and easy replacement of the sensors,
- allows a direct connection with a MAC address,
- does not require pre-connection discovery, thereby improving the performance of the wireless connectivity,
- is economical and cost-effective to implement, and
- is cost effective from both a manufacturer's and consumer's point of view.

These and other objects and advantages of the present invention will become apparent from the sub sequent detailed description of the preferred embodiment and the appended claims taken in conjunction with the accompanying drawings.

### BRIEF DESCRIPION OF THE DRAWINGS

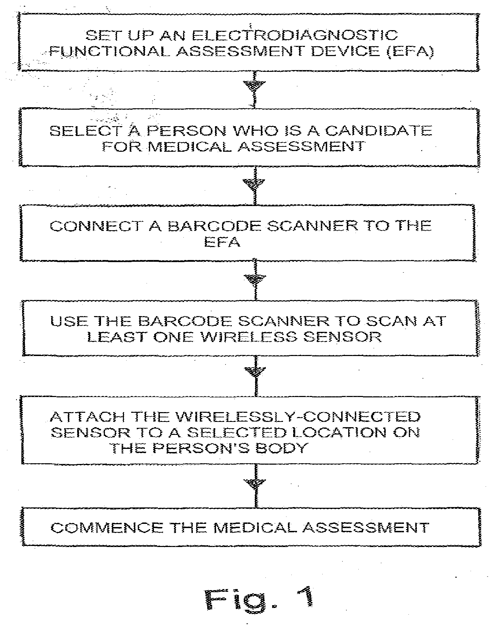

FIGURE 1 is a flow diagram showing the steps that are performed in the method.

### BEST MODE FOR CARRYING OUT THE INVENTION

The best mode for carrying out the invention is presented in terms that disclose a preferred embodiment of a system and method for establishing a wireless connection. Current conventional wireless connection means are accomplished by the use of technology that has remained substantially the same as when it was initially developed. A predominant function of conventional wireless connectivity is known as pairing, which is utilized by the BLUETOOTH® method of wireless data transmission. Pairing requires a strict protocol for establishing a wireless connection, and has inherent limitations on the functionality of the wireless connection.

The instant system and method for establishing a wireless connection (hereinafter "system and method"), allows the use of current wireless data transmission protocols such as BLUETOOTH®, as well as others including ZigBee and WiFi, without the requirement of pairing.

The system and method can be utilized with any device that allows wireless connectivity. These devices include, but are not limited to: a desktop computer, a laptop computer, a tablet computer, a smartphone, a cell phone, a printer or a camera. For this disclosure a medical diagnostic unit, which is known as an Electrodiagnostic Functional Assessment Device (EFA), will be described. The EFA is typically comprised of a computer running a proprietary software program and at least one wireless sensor that is attached to a selected location on a person's body. It should be noted that in most typical medical assessments, multiple wireless sensors are utilized. The software program facilitates the EFA identifying and establishing a wireless connection with the at least one wireless sensor.

As previously disclosed, the identification and establishment of the wireless connection is accomplished without the use of pairing. Since pairing is not utilized, any type of pre-pairing is also not required.

The instant system and method utilizes the proprietary software program to provide the necessary commands that cause the wireless connection to be established. The software program instructs the EFA to identify each wireless sensor and, once identification has been conformed, to then establish each wireless connection. The identification and establishment of the wireless connection is accomplished by use of a barcode scanner that is interfaced with the EFA and which is utilized to scan each wireless sensor. The scanned sensor data is then used by EFA software program to facilitate the bi-directional wireless connection.

The EFA and each wireless sensor can be connected via a radio connection or a wired access point. Again, since conventional pairing is not utilized, the instant wireless connection is directly accomplished without the use of a pre-connection discovery function.

The method portion of the instant system and method, as shown in FIGURE 1, is accomplished by performing the following steps:
1. Set up au Electrodiagnostic Functional Assessment Device (EFA).
2. Select a person who is a candidate for medical assessment.
3. Connect a barcode scanner to the EFA.
4. Use the barcode scanner to scan at least one wireless sensor, which allows the EFA software to identify the scanned sensor and to establish a bi-directional wireless connection between the EFA and the sensor.
5. Attach the wirelessly-connected sensor to a selected location on the person's body.
6. Commence the medical assessment.

The instant system and method provides significant improvement over conventional pairing connections. The instant system and method is easier to use and requires less time to set up, can provide a higher level of security, and can even allow, for example when using the EFA with wireless sensors, a wirelessly-connected sensor to be removed and replaced with another wireless sensor while maintaining the wireless connection.

While the invention has been described in detail and pictorially shown in the accompanying drawings it is not to be limited to such details, since many changes and modification may be made to the invention without departing from the scope thereof. Hence, it is described to cover any and all modifications and forms which may come within the scope of the claims.

## Claims

1. A system for medical assessment comprising an electrodiagnostic functional assessment device, EFA, running a software program, a barcode scanner connected to the EFA and at least one wireless-enabled sensor configured to be attached to a selected location on a person's body, the barcode scanner being configured to scan the at least one wireless-enabled sensor, wherein the software program is configured to instruct, using the scanned sensor data from the barcode scanner, the EFA to identify the at least one scanned wireless-enabled sensor and, once identification has been conformed, then to establish a bi-directional wireless connection between the EFA and the at least one scanned wireless-enabled sensor without the use of pairing, wherein the at least one wireless-enabled sensor is configured to provide to said EFA physiological data from the person's body and quantitative quality of service data including packet loss and sensor connection data.

2. The system as specified in claim 1, wherein said EFA and at least one wireless sensor are wirelessly connected via a radio connection or a wired access point.

3. The system as specified in claim 1, wherein a wireless communication protocol between the EFA and the at least one wireless sensor is selected from the group consisting of Bluetooth, ZigBee and WiFi.

## Patentansprüche

1. System zur medizinischen Bewertung, umfassend eine Vorrichtung zur elektrodiagnostischen Funktionsbewertung, EFA, die ein Softwareprogramm ausführt, einen Strichcodeleser, der mit der EFA verbunden ist, und mindestens einen drahtlosfähigen Sensor, der zur Befestigung an einer ausgewählten Stelle auf dem Körper einer Person ausgelegt ist, wobei der Strichcodeleser zum Abfragen des mindestens einen drahtlosfähigen Sensors ausgelegt ist, wobei das Softwareprogramm so ausgelegt ist, dass es die EFA unter Verwendung der abgefragten Sensordaten vom Strichcodeleser anweist, den mindestens einen abgefragten drahtlosfähigen Sensor zu identifizieren und dann, sobald die Identifikation übereinstimmt, eine bidirektionale drahtlose Verbindung zwischen der EFA und dem mindestens einen abgefragten drahtlosfähigen Sensor ohne die Verwendung von Kopplung herzustellen, wobei der mindestens eine drahtlosfähige Sensor so ausgelegt ist, dass er physiologische Daten vom Körper der Person und quantitative Dienstqualitätsdaten, die Paketverlustsdaten und Sensorverbindungsdaten umfassen, für die EFA bereitstellt.

2. System nach Anspruch 1, wobei die EFA und der mindestens eine drahtlose Sensor drahtlos über eine Funkverbindung oder einen drahtgebundenen Zugangspunkt verbunden sind.

3. System nach Anspruch 1, wobei das Drahtloskommunikationsprotokoll zwischen der EFA und dem mindestens einen drahtlosen Sensor aus der Gruppe bestehend aus Bluetooth, ZigBee und WiFi ausgewählt ist.

## Revendications

1. Système pour une évaluation médicale comprenant un dispositif d'évaluation fonctionnelle par électrodiagnostic, EFA, exécutant un programme logiciel, un lecteur de code à barres relié à l'EFA et au moins un capteur pouvant fonctionner sans fil configuré pour être fixé à un emplacement sélectionné sur le corps d'une personne, le lecteur de code à barres étant configuré pour balayer l'au moins un capteur pouvant fonctionner sans fil, dans lequel le programme logiciel est configuré pour donner l'instruction, en utilisant les données de capteur balayées à partir du lecteur de code à barres, à l'EFA d'identifier l'au moins un capteur pouvant fonctionner sans fil balayé et, une fois que l'identification a été conforme, d'établir ensuite une connexion sans fil bidirectionnelle entre l'EFA et l'au moins un capteur pouvant fonctionner sans fil balayé sans l'utilisation d'un appariement, dans lequel l'au moins un capteur pouvant fonctionner sans fil est configuré pour fournir audit EFA des données physiologiques provenant du corps d'une personne et des données de qualité de service quantitatives comprenant des données de perte de paquets et de connexion de capteur.

2. Système selon la revendication 1, dans lequel ledit EFA et au moins un capteur sans fil sont reliés sans fil par l'intermédiaire d'une connexion radio ou d'un point d'accès câblé.

3. Système selon la revendication 1, dans lequel un protocole de communication sans fil entre l'EFA et l'au moins un capteur sans fil est sélectionné parmi le groupe consistant en Bluetooth, ZigBee et WiFi.
